# EUROPEAN PATENT SPECIFICATION

(11) **EP 3 377 049 B1**
(45) Date of publication and mention of the grant of the patent: **06.01.2021**
(21) Application number: 16865892.0
(22) Date of filing: 16.11.2016
(51) Int. Cl.: A61K 31/00, A61P 25/04, C07C 215/54

(54) **CRYSTALLINE FORMS OF TAPENTADOL SALTS AND PROCESS FOR PREPARATION THEREOF**
KRISTALLINE FORMEN VON TAPENTADOLSALZEN UND VERFAHREN ZUR HERSTELLUNG DAVON
FORMES CRISTALLINES DE SELS DE TAPENTADOL ET LEUR PROCÉDÉ DE PRÉPARATION

(30) Priority: 17.11.2015 IN 6191CH2015
(43) Date of publication of application: 26.09.2018
(73) Proprietor: MSN Laboratories Private Limited, Hyderabad, Telangana 502329 (IN)
(72) Inventor: THIRUMALAI RAJAN, Srinivasan, Hyderabad Telangana (IN); KISHORE KUMAR, Muppa, Hyderabad Telangana (IN); VENKATESH, Mummadi, Hyderabad Telangana (IN); PRANEETH KUMAR, Anugu, Hyderabad Telangana (IN)
(74) Representative: dompatent von Kreisler Selting Werner - Partnerschaft von Patent- und Rechtsanwälten mbB
(86) International application number: PCT/IN2016/000270
(87) International publication number: WO 2017/085734

(56) References cited:
- WO-A1-2012/051246
- WO-A1-2012/051246
- WO-A1-2014/108514
- CN-A- 103 553 940
- US-A1- 2011 071 120
- US-B2- 8 981 154

## Description

### Field of the invention:

The present invention relates to crystalline Tapentadol tartrate, a process for the preparation thereof, a pharmaceutical composition comprising said crystelline Tapentadol tartrate and said compound for use in the treatment of pain. The structural formula of Tapentadol salts is represented as follows:

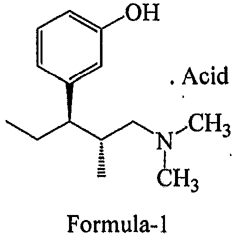

### Background of the invention:

Tapentadol HCl is chemically known as 3-[(1R,2R)-3-(dimethylamino)-1-ethyl-2-methylpropyl]phenol hydrochloride and is represented as the following structural formula.

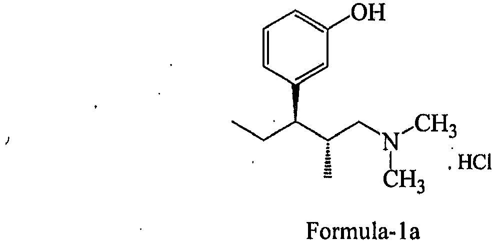

Tapentadol HCl is a centrally acting analgesic which is used in the management of pain. It has a dual mode of action, as an agonist at the µ-opioid receptor and as a norepinephrine reuptake inhibitor. Due to the dual mechanism of action as an opioid agonist and norepinephrine reuptake inhibitor, there is potential for off label use in chronic pain. In the US, 3-[(1R,2R)-3-(dimethylamino)-1-ethyl-2-methylpropyl]phenol hydrochloride is approved by FDA for the treatment of moderate to severe acute pain.

Tapentadol hydrochloride is commercially available as, immediate release dosage forms and as extended release dosage forms in the form of film coated tablets. These are currently marketed by Grunenthal under the brand name of Palexia® film coated tablets and Palexia® SR prolonged release tablets in Europe. In USA, Ortho McNeil Janssen markets it under the brand name of Nucynta® tablets and Nucynta® ER tablets.

Tapentadol hydrochloride, its synthesis, and its use as an analgesic are disclosed in European patent EP0693475 B1. Various processes for the preparation of tapentadol, its enantiomers and related compounds, and their pharmaceutically acceptable salts are disclosed in EP0693475 B1; US 6,248,737, US 6,344,558 and PCT patent publications WO 2004/108658, WO 2005/000788, WO 2008/012046, WO 2008/01.2047, WO2008/012283, WO2011080736, WO2011157390, WO20111026314, WO2013105109, WO2012089177 and also in a number of others.

The basic patent for tapentadol, EP0693475B1, discloses processes for the preparation of tapentadol or a pharmaceutically acceptable salt thereof. While it mentions that some of the disclosed compounds can Form salts with physiologically acceptable acids, for example, hydrochloric acid, hydrobromic acid, sulfuric acid, methanesulfonic acid, formic acid, acetic acid, oxalic acid, succinic acid, tartaric acid, mandelic acid, fumaric acid, lactic acid, citric acid, glutamic acid and/or aspartic acid, only the hydrochloride salt. However, out of the entire range of proposed salts it was only the crystalline hydrochloride that was prepared, isolated and sufficiently described. The polymorph of tapentadol hydrochloride isolated this way was identified as Form B.

European patents EP1612203 and EP 1799633 disclose polymorphic Forms A and B of tapentadol hydrochloride, and characterize them by powder X-ray diffraction (P-XR.D), Infra-Red spectroscopy (IR), Raman spectroscopy and crystal structure analysis. EP1612203 further teaches that the procedure described in example 25 of U.S. Pat. No. 6,248,737 and U.S. Pat. No. 6,344,558 as well as EP 0693475 B 1 produces crystalline Form B of tapentadol hydrochloride.

US2011071120 claims novel solid state Forms of tapentadol salts, process for their preparation, pharmaceutical compositions, and method of treating thereof. The tapentadol salts include an L-(-)-camphorsulfonate salt, a dibenzoyl-(L)-tartrate salt, a dibenzoyl-(D)-tartrate salt, a malate salt, a maleate salt, or a salicylate salt. However, salts with high molecular weight acids (e.g. camphor sulfonic, dibenzoyl tartaric acids and the like) are not suitable for pharmaceutical use as they may unacceptably increase the size of the dosage form (Handbook of Pharmaceutical Salts, Wiley, 2011, Chapter 7). This problem must be taken into account especially in the case of tapentadol as commercially available dosage forms contain 50 to 250 mg of tapentadol (as the hydrochloride).

A specific form of tapentadol hydrobromide was also described in WO2012051246. A wide range of salts and co-crystals is described in the application WO2012010316; however, most of the forms were not physically characterized in any way and no particular process of their preparation was mentioned. Only the preparation and isolation of tapentadol salts/co-crystals with (2S,3S)-dibenzoyl tartaric acid, sebacic acid, 1-hydroxy-2-naphthoic acid, embonic acid, nitric acid, nicotinic acid, hydrobromic acid, sulfuric acid, fumaric and malonic acid is disclosed in the examples. The said patent also discloses the PXRD of Tapentadol hemi-fumarate.

Polymorphs are distinct solids having the same molecular formula yet having distinct advantageous physical properties compared to other polymorphic forms of the same compound. The difference in the physical properties of different polymorphic forms results from the orientation and intermolecular interactions of adjacent molecules in the bulk solid.

Polymorphism, occurrence of different crystalline forms, is the property of some molecules and molecular complexes. It is generally known that various salts or polymorphs of pharmaceutically active substances may have different physico-chemical, and consequently pharmacological properties. Then; such salts and their polymorphs can be used to obtain an ideal composition of pharmaceutical formulations containing the given active substance or its salt. This means that it is very important to keep looking for suitable salts or polymorphic forms of pharmaceutically active substances. Discovering new salts and polymorphic forms of tapentadol may provide new methods of improving the characteristics of tapentadol as the active pharmaceutical component of formulations.

There remains a need for novel salts and novel solid state forms of tapentadol salts. The discovery of new polymorphic form of a pharmaceutically useful compound provides a new opportunity to improve the performance characteristics of a pharmaceutical product.

### Brief description of the invention:

The present invention and its preferred embodiments are apparent from the appendant set of claims.

### Brief description of the drawings:

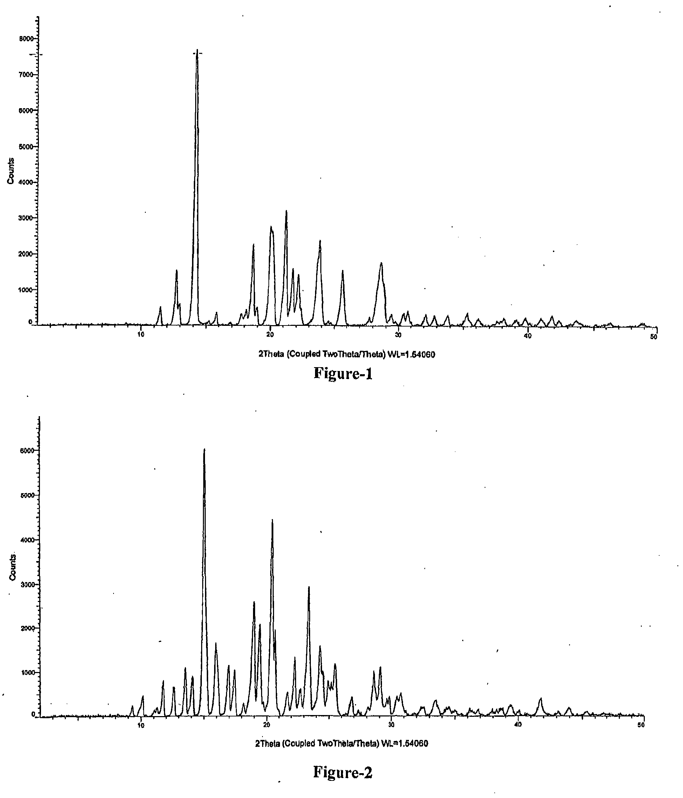

**Figure 1****:** Illustrates the PXRD pattern of crystalline form-M of Tapentadol tartrate
**Figure 2****:** Illustrates the PXRD pattern of crystalline form-S of Tapentadol fumarate.

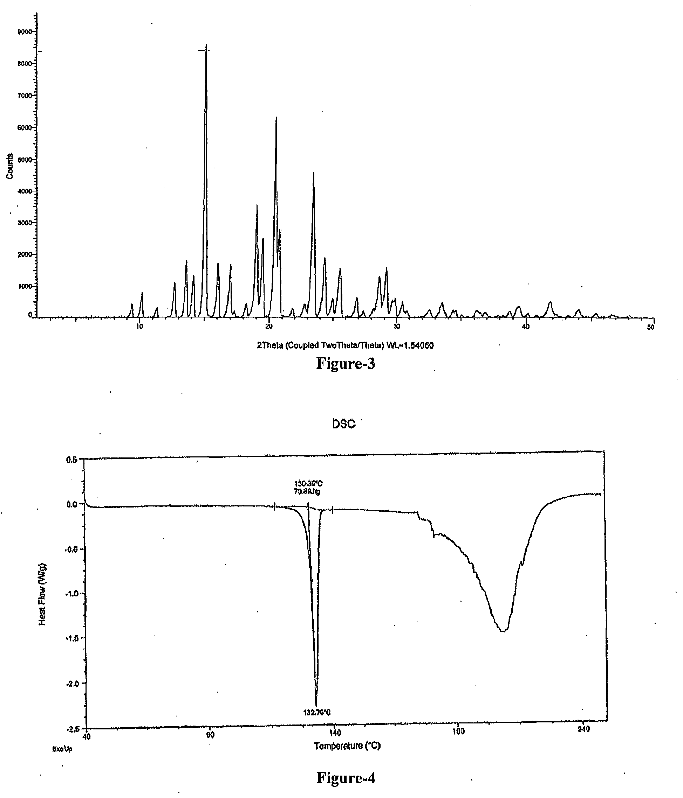

**Figure 3****:** Illustrates the PXRD pattern of crystalline form-N of Tapentadol fumarate
**Figure 4****:** Illustrates the DSC thermogram of crystalline form-M of Tapentadol tartrate.

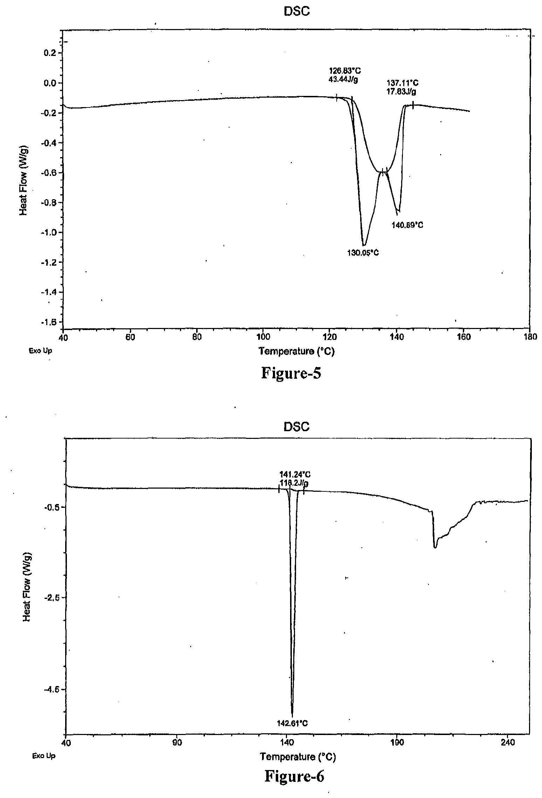

**Figure 5****:** Illustrates the DSC thermogram of crystalline form-S of Tapentadol fumarate.
**Figure 6****:** Illustrates the DSC thermogram of crystalline form-N of Tapentadol fumarate.

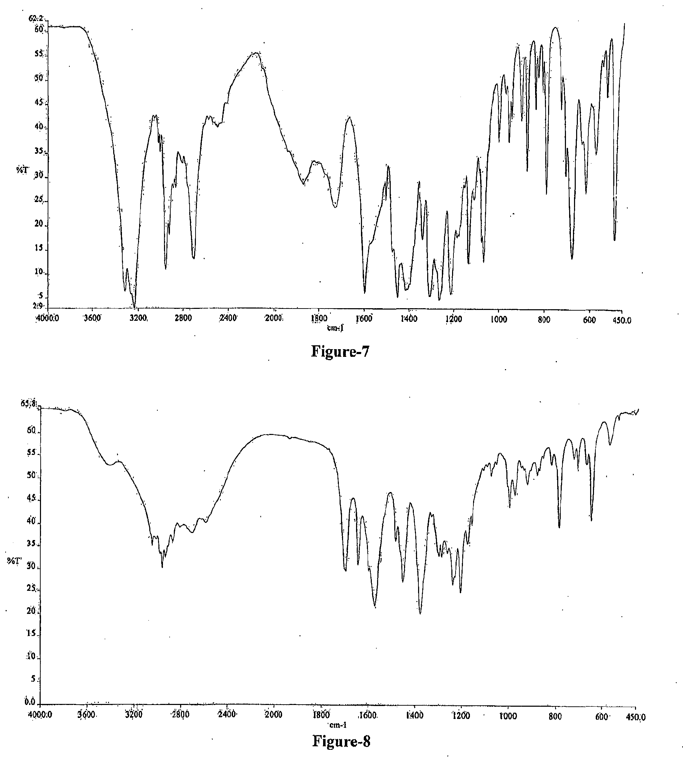

**Figure 7****:** Illustrates the IR spectrum of crystalline form-M of Tapentadol tartrate.
**Figure 8****:** Illustrates the IR spectrum of crystalline form-S of Tapentadol fumarate.

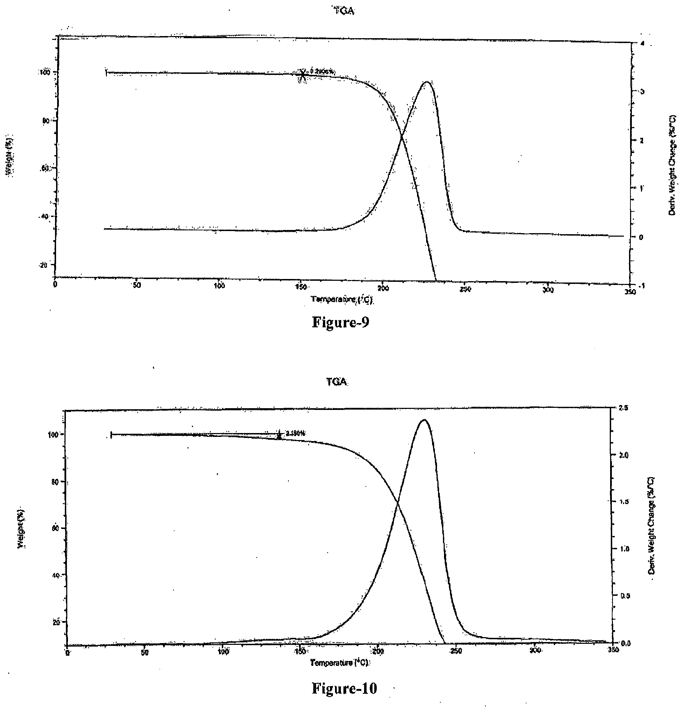

**Figure 9****:** Illustrates the TGA of crystalline form-M of Tapentadol tartrate.
**Figure 10****:** Illustrates the TGA of crystalline form-S of Tapentadol fumarate.

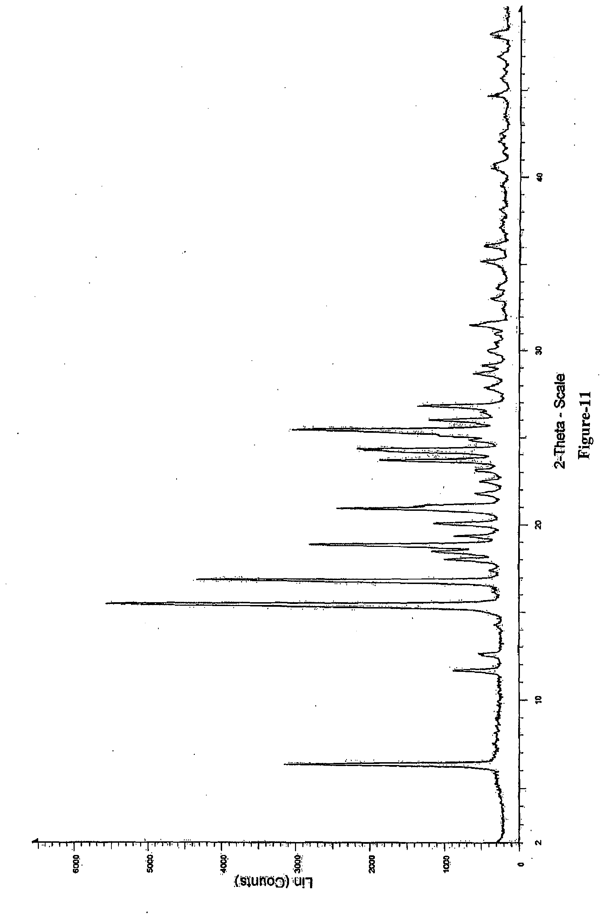

**Figure 11****:** Illustrates the PXRD pattern of crystalline form-L of Tapentadol bisulfate.

### Detailed description of the invention:

As used herein the term "suitable solvent" used in the present invention refers to "hydrocarbon solvents" such as n-hexane, n-heptane, cyclohexane, pet ether, benzene, toluene, pentane, cycloheptane, methyl cyclohexane, ethylbenzene, m-, o-, or p-xylene, or naphthalene and the like; "ether solvents" such as dimethoxymethane, tetrahydrofuran, 1,3-dioxane, 1,4-dioxane, furan, diethyl ether, ethylene glycol dimethyl ether, ethylene glycol diethyl ether, diethylene glycol dimethyl ether, diethylene glycol diethyl ether, triethylene glycol dimethyl ether, anisole, t-butyl methyl ether, 1,2-dimethoxy ethane and the like; "ester solvents" such as methyl acetate, ethyl acetate, isopropyl acetate, n-butyl acetate and the like; "polar-aprotic solvents such as dimethylacetamide (DMA), dimethylformamide (DMF), dimethylsulfoxide (DMSO), N-methylpyrrolidone (NMP) and the like; "chloro solvents" such as dichloromethane, dichloroethane, chloroform, carbon tetrachloride and the like; "ketone solvents" such as acetone, methyl ethyl ketone, methyl isobutylketone and the like; "nitrile solvents" such as acetonitrile, propionitrile, isobutyronitrile and the like; "alcoholic solvents" such as methanol, ethanol, n-propanol, isopropanol, n-butanol, isobutanol, t-butanol, 2-nitroethanol, 2-fluoroethanol, 2,2,2-trifluoroethanol, ethylene glycol, 1,2-propanediol (propylene glycol), 2-methoxyethanol, 1,2-ethoxyethanol, diethylene glycol, 1, 2, or 3-pentanol, neo-pentyl alcohol, t-pentyl alcohol, diethylene glycol monoethyl ether, cyclohexanol, benzyl alcohol, phenol, or glycerol and the like; "polar solvents" such as water or mixtures thereof.

The term "acid" used in the present invention refers to inorganic acids selected from hydrochloric acid, hydrobromic acid, sulfuric acid, nitric acid, phosphoric acid; organic acids such as acetic acid, maleic acid, malic acid, tartaric acid, oxalic acid, trifluoroacetic acid, methane sulfonic acid, p-toluene sulfonic acid; chiral acids such as S-(+) mandelic acid, R-(-) mandelic acid, L-(+)tartaric acid, D-(-)tartaric acid, L-malic acid, D-malic acid, D-maleic acid, (-)-naproxen, (+)-naproxen, (1R)-(-)-camphor sulfonic acid, (IS)- (+)-camphor sulfonic acid (1R)-(+)-bromocamphor-10-sulfonic acid, (IS)-(-)-bromocamphor-10-sulfonic acid, (-)-Dibenzoyl-L-tartaric acid, (-)-Dibenzoyl-L- tartaricacid monohydrate, (+)-Dibenzoyl-D - tartaric acid, (+)-Dibenzoyl-D -tartaric acid monohydrate, (+)-dipara-tolyl-D-tataric acid, (-)-dipara-tolyl-L-tataricacid, L(-)- pyroglutamic acid, L(+)-pyroglutamic acid, (-)-lactic acid, L-lysine, D-lysine etc., and like.

The term "salts" used in the present invention refers to acid addition salts selected from inorganic acids such as hydrochloric acid, hydrobromic acid, sulfuric acid, nitric acid, phosphoric acid; organic acids such as acetic acid, maleic acid, malic acid, oxalic acid, trifluoroacetic acid, methane sulfonic acid, p-toluene sulfonic acid; chiral acids such as S-(+) mandelic acid, R-(-) mandelic acid, L-(+)tartaric acid, D-(-)tartaric acid, L-malic acid, D-malic acid, D-maleic acid, (-)-naproxen, (+)-naproxen, (IR)-(-)-camphor sulfonic acid, (IS)-(+)-camphor sulfonic acid (1R)-(+)-bromocamphor-10-sulfonic acid, (IS)-(-)-bromocamphor-10-sulfonic acid, (-)-Dibenzoyl-L-tartaric acid, (-)-Dibenzoyl-L- tartaricacid monohydrate, (+)-Dibenzoyl-D -tartaric acid, (+)-Dibenzoyl-D-tartaric acid monohydrate, (+)-dipara-tolyl-D-tataric acid, (-)-dipara-tolyl-L-tataricacid, L(-)- pyroglutamic acid, L(+)-pyroglutamic acid, (-)-lactic acid, L-lysine, D-lysine etc., and like.

The first aspect of the present invention provides crystalline form-M of Tapentadol tartrate compound of formula-lb. The crystalline form-M of the present invention is characterized by its powder X-Ray diffraction pattern having peaks at about 12.7, 14.1, 18.6, 20.0, 21.1, 21.6, 22.1, 23.7, 25.6 & 28.5± 0.2 degrees of 2-theta. The crystalline form-M is further characterized by the PXRD pattern as illustrated in figure-1, its differential scanning calorimetric (DSC) thermogram having an endotherm at 132.76°C±3°C as illustrated in figure-4 and is further characterized by absorption peaks at 3319, 3237, 2960, 1731, 1597, 1305, 1263, 1213, 791, 679 and 485 cm⁻¹ in its infrared spectrum as illustrated in figure-7.

The second aspect of the present invention provides a process for the preparation of Tapentadol tartrate comprising:
a) Treating the (2R, 3R)-3-(3-methoxyphenyl)-N,N,2-trimethylpentan-1-amine hydrochloride with aqueous hydrogen bromide solution to provide tapentadol free base,
b) optionally isolating tapentadol free base,
c) treating tapentadol free base obtained in step-a) or step-b) with tartaric acid in a suitable solvent to provide Tapentadol tartrate,
d) optionally purifying the obtained compound.
Wherein, in step-c) the suitable solvent is selected from alcohol solvents, ketone solvents, ester solvents, hydrocarbon solvents, ether solvents, chloro solvents and mixture thereof or optionally in combination with water; preferably ketone solvents and most preferably acetone.

Preferred embodiment of the present invention provides a process for the preparation of crystalline form-M of Tapentadol tartrate compound of formula-1b comprising: 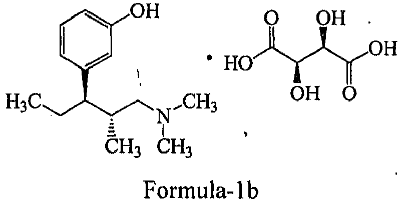
a) Treating the (2R, 3R)-3-(3-methoxyphenyl)-N,N,2-trimethylpentan-1-amine hydrochloride with aqueous hydrogen bromide solution to provide tapentadol free base,
b) treating tapentadol free base in-situ with L-(+)tartaric acid in acetone to produce crystalline form-M of Tapentadol tartrate.

Further embodiment of the present invention provides a process for the preparation of Tapentadol tartrate comprising; treating tapentadol free base with tartaric acid in a suitable solvent to provide Tapentadol tartrate; wherein the suitable solvent is selected from alcohol solvents, ketone solvents, ester solvents, hydrocarbon solvents, ether solvents, chloro solvents and mixture thereof or optionally in combination with water; preferably ketone solvents and most preferably acetone.

Preferred embodiment of the present invention provides a process for the preparation of crystalline form-M of Tapentadol tartrate comprising of; treating tapentadol free base with L-(+)tartaric acid in acetone to provide crystalline Tapentadol tartrate.

The crystalline Tapentadol tartrate of the present invention may have a purity of > 99 % by HPLC.
The present invention also relates to a pharmaceutical composition comprising crystalline Tapentadol tartrate and a pharmaceutically acceptable carrier.
The present invention also relates to crystalline Tapentadol tartarte in a pharmaceutical composition for use in the treatment of pain.

An aspect of the present disclosure provides crystalline form-S of Tapentadol fumarate. The crystalline form-S of the present invention is characterized by its powder X-Ray diffraction pattern having peaks at about 9.3, 11.7, 12.5, 13.5, 15.0, 18.1, 20.4, 20.5, 22.2, 22.7, & 29.0± 0.2 degrees of 2-theta. The crystalline form-S is further characterized by its PXRD pattern as illustrated in figure-2, its differential scanning calorimetric (DSC) thermogram having two endotherms at 130.05°C±3°C & 140.9°C±3°C as illustrated in figure-5 and is further characterized by its IR absorption spectrum having absorption peaks at 3050, 2965, 1698, 1644, 1573, 1506, 1453, 1379, 1239, 1206, 998, 977, 789, 652 and 573 cm⁻¹ as illustrated in figure-8.

A further aspect of the present disclosure provides crystalline form-N of Tapentadol fumarate. The crystalline form-N of the present invention is characterized by its powder X-Ray diffraction pattern having peaks at about 9.3, 12.5, 13.5, 15.0, 18.1, 20.4, 20.5, 22.7, & 29.0± 0.2 degrees of 2-theta. The crystalline form-N is further characterized by its PXRD pattern as illustrated in figure-3 and its differential scanning calorimetric (DSC) thermogram having an endotherm at 142.50°C±3°C as illustrated in figure-6.

An aspect of the present disclosure is a process for the preparation of Tapentadol fumarate compound of formula-1c comprising of: 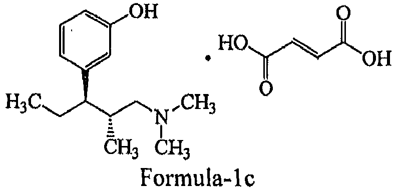
a) Treating the (2*R*, 3*R*)-3-(3-methoxyphenyl)-*N,N*,2-trimethylpentan-1-amine hydrochloride with aqueous hydrogen bromide solution to provide tapentadol free base,
b) optionally isolating tapentadol free base,
c) treating tapentadol free base obtained in step-a) or step-b) with fumaric acid in a suitable solvent to provide Tapentadol fumarate,
d) optionally purifying the obtained compound.
Wherein, in step-c) suitable solvent is selected from alcohol solvents, ketone solvents, ester solvents, hydrocarbon solvents, ether solvents, chloro solvents and mixture thereof or optionally in combination with water; preferably ketone solvents and most preferably acetone.

A preferred embodiment of the above-disclosed process for the preparation of crystalline Tapentadol fumarate comprises:
a) Treating the (2*R*,3*R*)-3-(3-methoxyphenyl)-*N*,*N*,2-trimethylpentan-1-amine hydrochloride with aqueous hydrogen bromide to provide tapentadol free base,
b) treating tapentadol free base in-situ with fumaric acid in acetone to provide crystalline Tapentadol fumarate.

Further disclosed is a process for the preparation of Tapentadol fumarate comprising of; treating Tapentadol free base with fumaric acid in a suitable solvent to provide Tapentadol fumarate; wherein the suitable solvent is selected from alcohol solvents, ketone solvents, ester solvents, hydrocarbon solvents, ether solvents, chloro solvents and mixture thereof or optionally in combination with water; preferably ketone solvents and most preferably acetone.

Another aspect of the description is a process for the preparation of crystalline Tapentadol fumarate comprising; treating Tapentadol free base with fumaric acid in acetone to provide crystalline Tapentadol fumarate.

Another aspect of the description provides crystalline form-L of Tapentadol bisulfate. The crystalline form-L is characterized by its powder X-Ray diffraction pattern having peaks at about 6.3,11.6, 15.3, 16.7, 18.8, 20.9, 23.6, 24.2, 25.3 and 26.7± 0.2 degrees of 2-theta and is further characterized by its PXRD pattern as illustrated in figure-11.

Disclosed is also a process for the preparation of crystalline Tapentadol bisulfate comprising; treating Tapentadol free base with sulfuric acid in a suitable solvent to provide crystalline Tapentadol bisulfate; wherein the suitable solvent is selected from alcohol solvents, ketone solvents, ester solvents, hydrocarbon solvents, ether solvents, chloro solvents and mixture thereof or optionally in combination with water.

Another aspect of the description is a process for the preparation of crystalline form-L of Tapentadol bisulfate compound of formula-1d comprising: 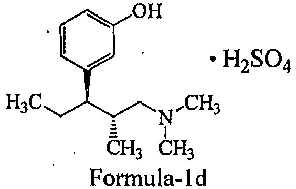 treating Tapentadol free base with sulfuric acid in ethanol to provide crystalline form-L of Tapentadol bisulfate.

The (2*R*,3*R*)-3-(3-methoxyphenyl)-*N,N*,2-trimethylpentan-1-amine hydrochloride and Tapentadol free base are prepared by the known prior art processes.

Crystalline forms of Tapentadol tartrate of the present invention can be utilized in the preparation of pharmaceutical composition useful for the treatment of pain.

PXRD analysis of crystalline forms of Tapentadol tartrate, tapentadol bisulfate & tapentadol fumarate were carried out using Bruker-AXS/ D8 advance X-Ray diffractometer using Cu Kα1, radiation of wavelength 1.5406 A° and continuous scan speed of 0.03°/min.

IR spectra were recorded on a Perkin-Elmer FTIR spectrometer.

Crystalline Tapentadol tartrate produced by the present invention as well as its bisulfate and fumarate salts can be further micronized or milled using conventional techniques to get the desired particle size to achieve desired solubility profile based on different forms of pharmaceutical composition requirements. Techniques that may be used for particle size reduction include, but not limited to ball, roller and hammer mills, and jet mills. Milling or micronization may be performed before drying, or after the completion of drying of the product.

The best mode of carrying out the present invention was illustrated by the below mentioned examples. These examples are provided as illustration only.

### Examples:

### Example-1: Preparation of crystalline form-M of Tapentadol tartrate

**Step-a:** Aqueous hydrogen bromide (400 ml) was added to (2*R*,3*R*)-3-(3-methoxyphenyl)-N,N,2-tri methylpentan-1-amine hydrochloride (100 gm) at 25-30°C. Heated the reaction mixture to reflux temperature and stirred for 1 ½ hour at the same temperature. Cooled the reaction mixture to 40-45°C and water was added to it. Further cooled the reaction mixture to 0-5°C. Basified the reaction mixture using aqueous ammonia at the same temperature. Ethyl acetate was added to the reaction mixture at the same temperature and raised the temperature of the reaction mixture to 25-30°C. Stirred the reaction mixture for 10 minutes and separated the both organic and aqueous layers. Distilled off the solvent from the organic layer under reduced pressure. Acetone (234 ml) was added to the obtained compound at 25-30°C and stirred for 10 minutes. Filtered the reaction mixture and washed with acetone.

**Step-b:** L(+)-tartarlc acid (52.87 gm) was dissolved in 780 ml of acetone at 50-55°C, filtered the obtained solution and washed with acetone. To the obtained reaction mixture added filtrate obtained in step-a) at 25-30°C. Stirred the reaction mixture for 1 ½ hour at 25-30°C. Filtered the precipitated solid, washed with acetone and dried to get the title compound.
Yield: 100 gm; Purity by RS/ HPLC: 99.94% and chiral purity by HPLC: 99.01%.
The PXRD of the obtained compound was shown in figure-1 and it's DSC in figure-4.

### Example-2: Preparation of crystalline form-S of Tapentadol fumarate (not according to the present invention) invention)

**Step-a:** Aqueous hydrogen bromide (400 ml) was added to (2*R*,3*R*)-3-(3-methoxyphenyl)-*N*,*N*,2-trimethylpentan-1-amine hydrochloride (100 gm) at 25-30°C. Heated the reaction mixture to reflux temperature and stirred for 1 ½ hour at the same temperature. Cooled the reaction mixture to 40-45°C and water was added to it. Further cooled the reaction mixture to 0-5°C. Basified the reaction mixture using aqueous ammonia at the same temperature. Ethyl acetate was added to the reaction mixture at the same temperature and raised the temperature of the reaction mixture to 25-30°C. Stirred the reaction mixture for 10 minutes and separated the both organic and aqueous layers. Distilled off the solvent from the organic layer under reduced pressure. Acetone (234 ml) was added to the obtained compound at 25-30°C and stirred for 10 minutes. Filtered the reaction mixture and washed with acetone.
Step-b: Fumaric acid (40.90 gm) was dissolved in 2886 ml of acetone at 50-55°C, filtered the obtained solution and washed with acetone. Distilled off the solvent completely under reduced pressure and acetone (546 ml) was added to it at 25-30°C. To the obtained reaction mixture added the filtrate obtained in step-a) at 25-30°C. Stirred the reaction mixture for 45 minutes at 25-30°C. Cooled the reaction mixture to 0-5°C and stirred for 1 hour at the same temperature. Filtered the precipitated solid, washed with acetone and dried to get the title compound. Yield: 110 gm; Purity by HPLC: 99.97% and chiral purity by HPLC: 99.02%. The PXRD of the obtained compound is shown in figure-2 and it's DSC in figure-5.

### Example-3: Preparation of crystalline form-M of Tapentadol tartrate

Tartaric acid (67.82 gm) was dissolved in 1000 ml of acetone at 50-55°C, filtered the obtained solution and washed with acetone. To the obtained reaction mixture added a solution of Tapentadol free base (100 gm) in acetone (300 ml) at 25-30°C. 0.1 gm of crystalline tapentadol tartrate was added to the reaction mixture at 25-30°C and stirred the reaction mixture for 1 ½ hour at the same temperature, Filtered the precipitated solid, washed with acetone and dried to get the title compound. Yield: 150 gm.
The PXRD pattern of the obtained compound is similar to figure-1.

### (not according to the Example-4: Preparation of crystalline form-N of Tapentadol fumarate present invention)

Fumaric acid (52.44 gm) was dissolved in 3500 ml of acetone at 50-55°C, filtered the obtained solution and washed with acetone. Distilled off the solvent completely under reduced pressure and acetone (700 ml) was added to it at 25-30°C. To the obtained reaction mixture added a solution of tapentadol free base (100 gm) in acetone (300 ml) at 25-30°C and stirred for 10 minutes. 0.1 gm of crystalline tapentadol fumarate was added to the reaction mixture at 25-30°C and stirred for 45 minutes at the same temperature. Cooled the reaction mixture to 0-5°C and stirred for 1 hour at the same temperature. Filtered the precipitated solid, washed with acetone and dried to get the title compound. Yield: 142 gm. The PXRD of the obtained compound is shown in figure-3 and it's DSC in figure-6.

### Example-5: Preparation of crystalline form-M of Tapentadol tartrate

**Step-a:** Aqueous hydrogen bromide (400 ml) was added to (2*R*,3*R*)-3-(3-methoxyphenyl)-N,N,2-tri methylpentan-1-amine hydrochloride (200 gm) at 25-30°C. Heated the reaction mixture to 115-120°C and stirred for 1 ½ hour at the same temperature. Cooled the reaction mixture to 40-45°C and water was added to it. Further cooled the reaction mixture to 0-5°C. Basified the reaction mixture using aqueous ammonia at the same temperature. Ethyl acetate was added to the reaction mixture at the same temperature and raised the temperature of the reaction mixture to 25-30°C. Stirred the reaction mixture for 10 minutes and separated both the organic and aqueous layers. The aqueous layer was extracted with ethyl acetate. Combined the organic layers and washed with aqueous sodium chloride solution followed by water. The organic was dried over sodium sulfate and carbon was added to the organic layer at 25-30°C. Stirred the reaction mixture for 45 minutes and filtered the reaction mixture. Distilled off the solvent completely from the filtrate under reduced pressure and co-distilled with acetone. Acetone (480 ml) was added to the obtained compound at 25-30°C and stirred for 10 minutes. Filtered the reaction mixture and washed with acetone.

**Step-b:** L(+)-tartaric acid (108.5 gm) was dissolved in 2400 ml of acetone at 50-55°C, filtered the obtained solution and washed with acetone. To the obtained reaction mixture added filtrate obtained in step-a) at 25-30°C and stirred the reaction mixture for 15 minutes at the same temperature. The reaction mixture was seeded with crystalline form-M of Tapentadol tartrate at 25-30°C and stirred the reaction mixture for 6 hours at the same temperature. Cooled the reaction mixture to 0-5°C and stirred it for 2 hours at the same temperature. Filtered the precipitated solid, washed with acetone and dried to get the title compound. Yield: 210 gm; Purity by RS/HPLC: 99.96%; PXRD of the obtained compound is shown to the figure-1. Tartaric acid content by Potentiometry is 40.14% w/w.
Particle size:- D(0.1): 3.822 µm, D(0.5): 25.472 µm, D(0.9): 95.404 µm.

### (not according to the Example-6: Preparation of crystalline form-S of Tapentadol fumarate present invention)

**Step-a:** Aqueous hydrogen bromide (400 ml) was added to (2*R*,3*R*)-3-(3-methoxyphenyl)-*N,N*,2-tri methylpentan-1-amine hydrochloride (200 gm) at 25-30°C. Heated the reaction mixture to 115-120°C and stirred for 1 ½ hour at the same temperature. Cooled the reaction mixture to 40-45°C and water was added to it. Further cooled the reaction mixture to 0-5°C. Basified the reaction mixture using aqueous ammonia at the same temperature. Ethyl acetate was added to the reaction mixture at the same temperature and raised the temperature of the reaction mixture to 25-30°C. Stirred the reaction mixture for 10 minutes and separated both the organic and aqueous layers. The aqueous layer was extracted with ethyl acetate. Combined the organic layers and washed with aqueous sodium chloride solution followed by water. The organic was dried over sodium sulfate and carbon was added to the organic layer at 25-30°C. Stirred the reaction mixture for 45 minutes and filtered the reaction mixture. Distilled off the solvent completely from the filtrate under reduced pressure and co-distilled with acetone. Acetone (480 ml) was added to the obtained compound at 25-30°C and stirred for 10 minutes. Filtered the reaction mixture and washed with acetone.

**Step-b:** Fumaric acid (83.88 gm) was.dissolved in 5920 ml of acetone at 50-55°C, filtered the obtained solution and washed with acetone. Distilled off the solvent completely from the filtrate under reduced pressure. 1120 ml of acetone was added to the obtained compound at 25-30°C and stirred for 5 minutes at the same temperature. To the obtained reaction mixture added filtrate obtained in step-a) at 25-30°C and stirred the reaction mixture for 15 minutes at the same temperature. The reaction mixture was seeded with crystalline form-S of Tapentadol fumarate at 25-30°C and stirred the reaction mixture for 35 minutes at the same temperature. Cooled the reaction mixture to 0-5°C and stirred it for 1 hour at the same temperature. Filtered the precipitated solid, washed with acetone and dried to get the title compound.
Yield: 220 gm; Purity by RS/HPLC: 99.92%. Fumaric acid content by Potentiometry is 37.66% w/w. PXRD of the obtained compound is shown in figure-2.

### Example-7: Preparation of crystalline form-L of Tapentadol bisulfate (not according to the present invention)

Dissolved 5 gms of tapentadol free base in 15 ml of ethanol at 25-30°C. Sulfuric acid (2.2 gm) was slowly added to the reaction mixture at 25-30° and stirred the reaction mixture for 45 minutes at the same temperature. Distilled off the solvent from the reaction mixture completely under reduced pressure and co-distilled with n-heptane. The obtained compound was kept a side for 3 days at 25-30°C, collected the solidified compound and dried to get the title compound.
Yield: 4 gm; PXRD of the obtained compound is shown in figure-11.

### Example-8: Preparation of Tapentadol tartrate

**Step-a:** Aqueous hydrogen bromide (200 ml) was added to (2*R*,3*R*)-3-(3-methoxyphenyl)-*N*,*N*,2-trimethylpentan-1-amine hydrochloride (100 gm) at 25-30°C. Heated the reaction mixture to 115-120°C and stirred for 1 ½ hour at the same temperature. Cooled the reaction mixture to 40-45°C and water was added to it. Further cooled the reaction mixture to 0-5°C. Basified the reaction mixture using aqueous ammonia at the same temperature. Ethyl acetate was added to the reaction mixture at the same temperature and raised the temperature of the reaction mixture to 25-30°C. Stirred the reaction mixture for 10 minutes and separated both the organic and aqueous layers. The aqueous layer was extracted with ethyl acetate. Combined the organic layers and washed with water. The organic was dried over sodium sulfate. Distilled off the solvent completely from the filtrate under reduced pressure. Methyl tertiarybutyl ether (390 ml) was added to the obtained compound at 25-30°C and stirred for 10 minutes. Filtered the reaction mixture and washed with methyl tertiarybutyl ether. **Step-b:** L(+)-tartaric acid (52.87 gm) was dissolved in 390 ml of isopropyl alcohol at 43-48°C, filtered the obtained solution and washed with isopropyl alcohol. Heated the obtained filtrate to 43-48°C and slowly added filtrate obtained in step-a) at the same temperature stirred the reaction mixture for 15 minutes at the same temperature. The reaction mixture was seeded with crystalline Tapentadol tartrate at 40-43°C. Cooled the reaction mixture to 25-30°C and stirred for 45 minutes at the same temperature. Further cooled the reaction mixture to -10 to -5°C and stirred it for 2 hours at the same temperature. Filtered the precipitated solid, washed with the mixture of isopropanol & methyl tertiarybutyl ether and dried to get the title compound. Yield: 105 gm.

## Claims

1. Crystalline Tapentadol tartrate compound of formula-1b

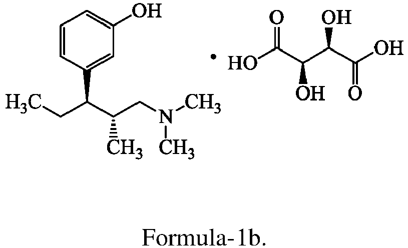

2. The crystalline Tapentadol tartrate according to claim 1, wherein said crystalline form is **characterized by** its powder X-Ray diffraction pattern having peaks at 12.7, 14.1, 18.6, 20.0, 21.1, 21.6, 22.1, 23.7, 25.6 and 28.5± 0.2 degrees of 2-theta.

3. The crystalline Tapentadol tartrate according to claim 2, is having the endotherm at 132°C±3°C in its differential scanning calorimetric (DSC) thermogram.

4. The crystalline Tapentadol tartrate according to claim 2 or 3, **characterized by** absorption peaks at 3319, 3237, 2960, 1731, 1597, 1305, 1263, 1213, 791, 679 and 485 cm⁻¹ in its infrared spectrum.

5. The crystalline Tapentadol tartrate according to any one of claims 1 to 4, wherein the crystalline Tapentadol tartrate has a purity of >99 % by HPLC.

6. A process for the preparation of the crystalline Tapentadol tartrate as defined in claim 1, said process comprising the step of: treating tapentadol free base with tartaric acid in a suitable solvent to provide Tapentadol tartrate of formula-1b.

7. The process according to claim 6, said process comprising the steps of:
a) treating the (2R,3R)-3-(3-methoxyphenyl)-N,N,2-trimethylpentan-1-amine hydrochloride with aqueous hydrogen bromide solution to provide tapentadol free base,
b) optionally isolating tapentadol free base,
c) treating tapentadol free base obtained in step-a) or step-b) with tartaric acid in a suitable solvent to provide Tapentadol tartrate of formula-1b,
d) optionally purifying the compound obtained in step-c) from a suitable solvent.

8. A process for the preparation of crystalline Tapentadol tartrate as defined in claim 2, said process comprising the step of: treating tapentadol free base with L-(+)tartaric acid in acetone to provide the crystalline Tapentadol tartrate of formula-1b.

9. The process for the preparation of crystalline Tapentadol tartrate according to claim 8, said process comprising the steps of:
a) treating the (2R,3R)-3-(3-methoxyphenyl)-N,N,2-trimethylpentan-1-amine hydrochloride with aqueous hydrogen bromide solution to provide tapentadol free base,
b) treating tapentadol free base in-situ with L-(+)tartaric acid in acetone to provide the crystalline Tapentadol tartrate compound of formula-1b.

10. The process according to any one of claims 6 and 7, wherein the suitable solvent is selected from alcohol solvents, ketone solvents, ester solvents, hydrocarbon solvents, ether solvents, chloro solvents and mixtures thereof or optionally in combination with water.

11. The crystalline Tapentadol tartrate as defined in any one of claims 2 to 5, in a pharmaceutical composition for use in the treatment of pain.

12. A pharmaceutical composition comprising the crystalline Tapentadol tartrate as defined in any one of claims 2 to 5, and a pharmaceutically acceptable carrier.

## Patentansprüche

1. Kristalline Tapentadoltartrat-Verbindung der Formel 1b:

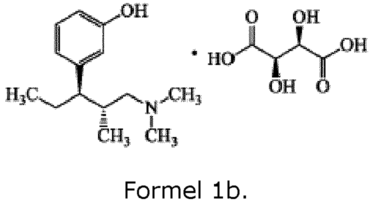

2. Kristallines Tapentadoltartrat gemäß Anspruch 1, wobei die kristalline Form durch ihr Pulverröntgenbeugungsmuster gekennzeichnet ist, das Reflexe bei 2 Theta von 12,7, 14,1, 18,6, 20,0, 21,1, 21,6, 22,1, 23,7, 25,6 und 28,5 ± 0,2 Grad aufweist.

3. Kristallines Tapentadoltartrat gemäß Anspruch 2, das im Thermogramm der dynamischen Differenzkalorimetrie (DSC) eine Endotherme bei 132 °C ± 3 °C aufweist.

4. Kristallines Tapentadoltartrat gemäß Anspruch 2 oder 3, **gekennzeichnet durch** Absorptionsmaxima bei 3319, 3237, 2960, 1731, 1597, 1305, 1263, 1213, 791, 679 und 485 cm⁻¹ im Infrarotspektrum.

5. Kristallines Tapentadoltartrat gemäß einem der Ansprüche 1 bis 4, wobei das kristalline Tapentadoltartrat eine durch HPLC bestimmte Reinheit von >99% aufweist.

6. Verfahren zur Herstellung des kristallinen Tapentadoltartrats gemäß Anspruch 1, wobei das Verfahren den Schritt des Behandelns der freien Base von Tapentadol mit Weinsäure in einem geeigneten Lösungsmittel unter Bildung des Tapentadoltartrats der Formel 1b umfasst.

7. Verfahren gemäß Anspruch 6, wobei das Verfahren die folgenden Schritte umfasst:
a) Behandeln des (2R,3R)-3-(3-Methoxyphenyl)-N,N,2-trimethylpentan-1-amin-Hydrochlorids mit einer wässrigen Bromwasserstoffsäure unter Bildung der freien Base von Tapentadol;
b) gegebenenfalls Isolieren der freien Base von Tapentadol;
c) Behandeln der in Schritt a) oder Schritt b) erhaltenen freien Base von Tapentadol mit Weinsäure in einem geeigneten Lösungsmittel unter Bildung des Tapentadoltartrats der Formel 1b;
d) gegebenenfalls Reinigen der in Schritt c) erhaltenen Verbindung aus einem geeigneten Lösungsmittel.

8. Verfahren zur Herstellung des kristallinen Tapentadoltartrats gemäß Anspruch 2, wobei das Verfahren den Schritt des Behandelns der freien Base von Tapentadol mit L-(+)-Weinsäure in Aceton unter Bildung des kristallinen Tapentadoltartrats der Formel 1b umfasst.

9. Verfahren zur Herstellung des kristallinen Tapentadoltartrats gemäß Anspruch 8, wobei das Verfahren die folgenden Schritte umfasst:
a) Behandeln des (2R,3R)-3-(3-Methoxyphenyl)-N,N,2-trimethylpentan-1-amin-Hydrochlorids mit einer wässrigen Bromwasserstoffsäure unter Bildung der freien Base von Tapentadol;
b) Behandeln der freien Base von Tapentadol in situ mit L-(+)-Weinsäure in Aceton unter Bildung der kristallinen Tapentadoltartrat-Verbindung der Formel 1b.

10. Verfahren gemäß einem der Ansprüche 6 und 7, wobei das geeignete Lösungsmittel aus Alkohol-Lösungsmitteln, Keton-Lösungsmitteln, Ester-Lösungsmitteln, Kohlenwasserstoff-Lösungsmitteln, Ether-Lösungsmitteln, chlorhaltigen Lösungsmitteln und Gemischen davon oder gegebenenfalls in Kombination mit Wasser ausgewählt ist.

11. Kristallines Tapentadoltartrat gemäß einem der Ansprüche 2 bis 5 in einer pharmazeutischen Zusammensetzung zur Verwendung bei der Behandlung von Schmerzen.

12. Pharmazeutische Zusammensetzung, die das kristalline Tapentadoltartrat gemäß einem der Ansprüche 2 bis 5 und einen pharmazeutisch annehmbaren Träger umfasst.

## Revendications

1. Composé tartrate de Tapentadol cristallin de formule-1b

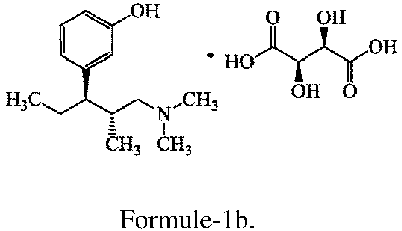

2. Tartrate de Tapentadol cristallin selon la revendication 1, où ladite forme cristalline est **caractérisée par** son diagramme de diffraction des rayons X de poudre ayant des pics à 12,7, 14,1, 18,6, 20,0, 21,1, 21,6, 22,1, 23,7, 25,6 et 28,5±0,2 degrés de 2-théta.

3. Tartrate de Tapentadol cristallin selon la revendication 2, dont l'endotherme est à 132°C±3°C dans son thermogramme calorimétrique différentiel à balayage (DSC).

4. Tartrate de Tapentadol cristallin selon la revendication 2 ou 3, caractérisé pas des pics d'absorption à 3319, 3237, 2960, 1731, 1597, 1305, 1263, 1213, 791, 679 et 485 cm⁻¹ dans son spectre infrarouge.

5. Tartrate de Tapentadol cristallin selon l'une quelconque des revendications 1 à 4, ledit tartrate de Tapentadol cristallin ayant une pureté de >99% par CLHP.

6. Procédé pour la préparation du tartrate de Tapentadol cristallin tel que défini dans la revendication 1, ledit procédé comprenant l'étape de : traitement de la base libre du tapentadol avec de l'acide tartrique dans un solvant approprié pour obtenir le tartrate de Tapentadol de formule-1b.

7. Procédé selon la revendication 6, ledit procédé comprenant les étapes de :
a) traitement du chlorhydrate de (2R,3R)-3-(3-méthoxyphényl)-N,N,2-triméthylpentan-1-amine avec une solution aqueuse de bromure d'hydrogène pour obtenir la base libre du tapentadol,
b) éventuellement, isolement de la base libre du tapentadol,
c) traitement de la base libre du tapentadol obtenue dans l'étape a) ou l'étape b) avec de l'acide tartrique dans un solvant approprié pour obtenir le tartrate de Tapentadol de formule-1b,
d) éventuellement, purification du composé obtenu dans l'étape c) à partir d'un solvant approprié.

8. Procédé pour la préparation du tartrate de Tapentadol cristallin tel que défini dans la revendication 2, ledit procédé comprenant l'étape de : traitement de la base libre du tapentadol avec de l'acide L-(+)tartrique dans de l'acétone pour obtenir le tartrate de Tapentadol cristallin de formule-1b.

9. Procédé pour la préparation du tartrate de Tapentadol cristallin selon la revendication 8, ledit procédé comprenant les étapes de :
a) traitement du chlorhydrate de (2R,3R)-3-(3-méthoxyphényl)-N,N,2-triméthylpentan-1-amine avec une solution aqueuse de bromure d'hydrogène pour obtenir la base libre du tapentadol,
b) traitement de la base libre du tapentadol in situ avec de l'acide L-(+)tartrique dans de l'acétone pour obtenir le composé tartrate de Tapentadol cristallin de formule-1b.

10. Procédé selon l'une quelconque des revendications 6 et 7, dans lequel le solvant approprié est choisi parmi les solvants alcooliques, les solvants cétoniques, les solvants ester, les solvants hydrocarbonés, les solvants éther, les solvants chlorés et leurs mélanges ou éventuellement en combinaison avec de l'eau.

11. Tartrate de Tapentadol cristallin tel que défini dans l'une quelconque des revendications 2 à 5, dans une composition pharmaceutique destinée à être utilisée dans le traitement de la douleur.

12. Composition pharmaceutique comprenant le tartrate de Tapentadol cristallin tel que défini dans l'une quelconque des revendications 2 à 5, et un véhicule pharmaceutiquement acceptable.
